# EUROPEAN PATENT SPECIFICATION

(11) **EP 3 212 198 B1**
(45) Date of publication and mention of the grant of the patent: **23.12.2020**
(21) Application number: 15855329.7
(22) Date of filing: 29.10.2015
(51) Int. Cl.: A61K 31/702, A61K 31/716, A61K 31/7004, A61K 31/7012, A61K 31/7016, A61P 1/04, A61K 35/20, A23L 33/125, A23L 33/00

(54) **SYNTHETIC COMPOSITION AND METHOD FOR PROMOTING MUCOSAL HEALING**
SYNTHETISCHE ZUSAMMENSETZUNG UND VERFAHREN ZUR FÖRDERUNG VON SCHLEIMHAUTWUNDHEILUNG
COMPOSITION SYNTHÉTIQUE ET PROCÉDÉ POUR FAVORISER LA CICATRISATION DES MUQUEUSES

(30) Priority: 29.10.2014 DK 201470662
(43) Date of publication of application: 06.09.2017
(73) Proprietor: Glycom A/S, 2970 Hørsholm (DK)
(72) Inventor: HENNET, Thierry, CH-8112 Otelfingen (CH); MCCONNELL, Bruce, CH-1814 La Tour de Peilz (CH); ELISON, Emma, S-24564 Hjärup (SE); VIGSNÆS, Louise Kristine, DK-2400 Copenhagen NV (DK)
(74) Representative: Inspicos P/S
(86) International application number: PCT/DK2015/050331
(87) International publication number: WO 2016/066174

(56) References cited:
- WO-A1-2011/005681
- WO-A1-2012/158517
- WO-A1-2013/016111
- WO-A1-2013/032674
- WO-A1-2013/154725
- WO-A2-2005/067962
- WO-A2-2012/106662
- WO-A2-2012/106665
- US-A1- 2012 171 165
- US-A1- 2012 171 166
- US-A1- 2012 172 319
- US-A1- 2012 172 327
- LARA-VILLOSLADA, F. ET AL.: 'Oligosaccharides isolated from goat mill reduce intestinal inflammation in a rat model of dextran sodium sulphate-induce colitis' CLINICAL NUTRITION vol. 25, 2006, pages 477 - 488, XP005492243
- PAPI, C. ET AL.: 'Mucosal healing in inflammatory bowel disease: Treatment efficacy and predictive factors' DIGESTIVE AND LIVER DISEASE vol. 45, 2013, pages 978 - 985, XP028765344

## Description

### FIELD OF THE INVENTION

This invention relates generally to synthetic compositions and methods for promoting mucosal healing in the gastrointestinal tract of humans.

### BACKGROUND TO THE INVENTION

The healing of the inflamed mucosa (mucosal healing) of humans is an emerging new goal for therapy for many inflammatory conditions in the gastrointestinal (GI) tract. Further, mucosal healing is increasingly viewed as a predictor of clinical remission in inflammatory conditions of the GI tract. This is especially the case with inflammatory bowel diseases (IBDs) such as Crohn's Disease (CD) and ulcerative colitis (UC). However it is equally applicable to many other inflammatory conditions of the GI tract where ulceration occurs such as chemotherapy induced ulceration in cancer patients.

IBDs are chronic relapsing diseases that lead to structural damage with destruction of the bowel wall (Baumgart et al. Lancet 380, 1590 (2012)). Generally, treatment of these diseases follows a stepwise approach where the first step is administration of 5-aminosalicylates, which are local acting anti-inflammatories. These agents appear to have greater efficacy for the treatment of ulcerative colitis than for Crohn's disease, for which efficacy data are limited.

If the patient's condition fails to respond to an adequate dose of 5-aminosalicylates, the second step is often corticosteroids, which tend to provide rapid relief of symptoms and a significant decrease in inflammation (Ford et al. Am. J. Gastroenterol. 106, 590 (2011)). In general, a major goal is to wean the patient off steroids as soon as possible to prevent long-term adverse effects from these agents. Most patients can tolerate a relatively rapid taper down in dose after a response is achieved. However occasionally, a very prolonged steroid taper is necessary to prevent relapse in patients who have had prolonged exposure to steroids in the past.

If oral corticosteroid therapy fails, the third step is usually immunomodulators or anti-TNF therapy (Papi et al. Dig. Liver Dis. 45, 978 (2013)). Anti-TNF monoclonal antibody therapies in particular are highly effective; at least initially. In particular, the anti-TNF-a antibodies adalimumab, certolizumab pegol, and infliximab are effective for induction and maintenance of remission in both CD and UC. However subgroups of patients do not respond to therapy and other subgroups develop neutralising antibodies. These patients show little or no change of clinical symptoms. Also all patients are exposed to side effects of this type of therapy, such as infections, reactivation of tuberculosis, allergic reactions, skin disorders, demyelinating disorders, and lupus-like autoimmunity. For this reason, these therapies are usually only given to severe patients; or moderate patients with a poor prognosis.

Until recently, most treatments focused on inducing clinical remission, reducing diarrhoea and reducing abdominal pain. However, mucosal healing, as visible by endoscopy, has recently emerged as a key treatment goal (Neurath et al. Gut 61, 1619 (2012)). Thus, the term "mucosal healing on endoscopy" has been developed to refer to visible resolution of ulcers in CD and erosions and ulcers in UC (Froslie et al. Gastroenterology 133, 412 (2007)). Mucosal healing has been associated with more effective disease control, more frequent steroid-free remission of disease, lower rates of hospitalization and surgery, and improved quality of life as compared with conventional treatment goals. These findings highlight the role of mucosal healing for therapy of IBD and inflammatory GI conditions in general.

Although mucosal healing by various drug therapies has been an appealing goal for IBD treatment (see Vaughn et al. Curr. Treat. Options Gastroenterol. 12, 103 (2014) and references therein), it has not always been achievable and has exposed some patients to unnecessary risks, particularly when it has led to escalating drug therapies. Attempts have therefore been made to treat patients having inflammatory gastrointestinal conditions with nutritional compositions; for example containing casein derived TGF-beta (US 5952295, WO 2014/020004), galactooligosaccharides (WO 2013/016111) or selected human milk oligosaccharides (WO 2013/032674). Nutritional compositions have offered the advantage of being safer and more suitable for long-term, chronic use. However, strong evidence of their efficacy has been lacking, and it has not been clear that they promote mucosal healing.

Therefore, there has remained a need for therapies which promote mucosal healing in inflammatory GI conditions of humans, and which are effective and safe with little or no adverse side effects.

### SUMMARY OF THE INVENTION

In one aspect, this invention provides a synthetic composition for use in:
- promoting mucosal healing in a patient, advantageously a human, having ulceration due to an inflammatory gastrointestinal condition,
- inducing mucosal healing in a patient, advantageously a human, having ulceration due to having an inflammatory gastrointestinal condition,
- treating a patient with IBD, preferably a patient with moderate-to-severe IBD who is receiving immunomodulator treatment, advantageously a human, having ulceration, to induce remission by inducing and/or promoting mucosal healing in the patient, and/or
- maintaining a patient with IBD, preferably a patient with moderate-to-severe IBD who is receiving immunomodulator treatment, advantageously a human, in remission by inducing/promoting mucosal healing in the patient and maintaining mucosal integrity,
characterised in that the synthetic composition contains an effective amount of one or more human milk monosaccharides or one or more human milk oligosaccharides or both. The synthetic composition is preferably a nutritional composition. The patient may be a paediatric or adult patient. The composition may further comprise a source of threonine, serine and/or proline. Preferably human milk oligosaccharides include both fucosyl and sialyl HMOs. The human milk oligosaccharides may also include backbone oligosaccharides such as LNT and LNnT.

In another aspect, this invention provides a method for
- inducing/promoting mucosal healing in a patient, advantageously a human, having ulceration due to having an inflammatory gastrointestinal condition,
- treating a patient with IBD, preferably a patient with moderate-to-severe IBD who is receiving immunomodulator treatment, advantageously a human, having ulceration, to induce remission by inducing and/or promoting mucosal healing in the patient, and/or
- maintaining a patient with IBD, preferably a patient with moderate-to-severe IBD who is receiving immunomodulator treatment, advantageously a human, in remission by inducing/promoting mucosal healing in the patient and maintaining mucosal integrity,
the method comprising orally administering to the patient an effective amount of one or human milk monosaccharides or one or more human milk oligosaccharides, or both, preferably in the form of a synthetic composition. The patient may be a paediatric or adult patient.

In a further aspect, this invention relates to a use of one or more human milk monosaccharides or one or more human milk oligosaccharides or both, preferably in the form of a synthetic composition, for
- inducing/promoting mucosal healing in a patient, advantageously a human, having ulceration due to an inflammatory gastrointestinal condition,
- treating a patient with IBD, preferably a patient with moderate-to-severe IBD who is receiving immunomodulator treatment, advantageously a human, having ulceration, to induce remission by inducing and/or promoting mucosal healing in the patient, and/or
- maintaining an IBD patient, preferably a patient with moderate-to-severe IBD who is receiving immunomodulator treatment, advantageously a human, in remission by inducing/promoting mucosal healing in the patient and maintaining mucosal integrity.

The patient may be a paediatric or adult patient.

It has been surprisingly found that human milk monosaccharides, advantageously sialic acid, and human milk oligosaccharides, advantageously 2'-FL, 3-FL, LNT, LNnT, 3'-SL, 6'-SL, DFL, DSLNT and/or LNFP-I not only modulate inflammation and microbiota in the GI tract, but also improve mucosal healing and help maintain mucosal integrity in human patients.

### DETAILED DESCRIPTION OF THE INVENTION

The term "oral administration" preferably means any conventional form for the oral delivery of a composition to a patient that causes the deposition of the composition in the gastrointestinal tract (including the stomach) of the patient. Accordingly, oral administration includes swallowing of composition by the patient, enteral feeding through a naso-gastric tube, and the like.

The term "effective amount" preferably means an amount of a composition that provides a human milk monosaccharide or human milk oligosaccharide in a sufficient amount to render a desired treatment outcome in a patient. An effective amount can be administered in one or more doses to the patient to achieve the desired treatment outcome.

The term "human milk monosaccharide" or "HMS" preferably means a monosaccharide found in human breast milk. Examples include sialic acid and L-fucose. In human milk, the sialic acid is N-acetylneuraminic acid.

The term "human milk oligosaccharide" or "HMO" preferably means a complex carbohydrate found in human breast milk that can be in acidic or neutral form. More than about 200 different HMO structures are known to exist in human breast milk (Urashima et al.: Milk Oligosaccharides, Nova Biomedical Books, New York, 2011). HMOs can be backbone, fucosylated and sialylated oligosaccharides. Backbone HMOs consists of Glu, Gal and GlcNAc and are devoid of Fuc and sialic acid. Examples of backbone HMOs include lacto-N-tetraose (LNT), lacto-N-neotetraose (LNnT), lacto-N-neohexaose (LNnH) and lacto-N-hexaose (LNH). Fucosyl HMOs are fucosylated lactoses or fucosylated backbone HMOs such as 2'-fucosyllactose (2'-FL), lacto-N-fucopentaose I(LNFP-I), lacto-N-difucohexaose I(LNDFH-I), 3-fucosyllactose (3-FL), difucosyllactose (DFL), lacto-N-fucopentaose III (LNFP-III), fucosyl-para-lacto-N-neohexaose (F-pLNnH), lacto-N-difucohexaose I (LNDFH-I), fucosyl-lacto-N-hexaose II (FLNH-II), lacto-N-fucopentaose V (LNFP-V), lacto-N-difucohexaose II (LNDFH-II), fucosyl-lacto-N-hexaose I (FLNH-I), fucosyl-lacto-N-hexaose III (FLNH-III) and fucosyl-para-lacto-N-neohexaose (F-pLNnH). Sialyl HMOs are sialylated lactoses or sialylated backbone HMOs such as 3',6-disialyllacto-N-tetraose (DSLNT), 6'-sialyllactose (6'-SL), 3'-sialyllactose (3'-SL), 6'-sialyllacto-N-neotetraose (LST c), 3'-sialyllacto-N-tetraose (LST a) and 6-sialyllacto-N-tetraose (LST b). HMOs containing both sialyl and fucosyl groups may be considered to belong to either of the latter two groups. Examples for sialyl and fucosyl HMOs include disialyl-fucosyl-lacto-N-hexaose II (DSFLNH-II), fucosyl-sialyl-lacto-N-neohexaose I (FSLNnH-I), fucosyl-sialyl-lacto-N-hexaose I (FSLNH-I) and 3-fucosyl-3'-sialyllactose (FSL).

The term "mucosal healing" preferably means a reduction in the number or concentration of visible ulcers on endoscopic assessment of an inflammatory gastrointestinal condition or an IBD. Various tests have been developed to assess mucosal healing. For example, for UC, tests such as the Ulcerative Colitis Colonoscopic Index of Severity (UCCIS) and the Ulcerative Colitis Endoscopic Index of Severity (UCEIS) can be used to assess mucosal healing. Similarly, for CD, tests such as the Crohn's Disease Endoscopic Index of Severity (CDEIS) and the Simplified Endoscopic Score - Crohn's Disease (SES-CD) can be used to assess mucosal healing. Diagnostic methods using biomarkers may also be used, for example as disclosed in WO 2014/054013.

The HMOs can be isolated or enriched by well-known processes from milk(s) secreted by mammals including, but not limited to human, bovine, ovine, porcine, or caprine species. The HMOs can also be produced by well-known processes using microbial fermentation, enzymatic processes, chemical synthesis, or combinations of these technologies. As examples, using chemistry LNnT can be made as described in WO 2011/100980 and WO 2013/044928, LNT can be synthesized as described in WO 2012/155916 and WO 2013/044928, a mixture of LNT and LNnT can be made as described in WO 2013/091660, 2'-FL can be made as described in WO 2010/115934 and WO 2010/115935, 3-FL can be made as described in WO 2013/139344, 6'-SL and salts thereof can be made as described in WO 2010/100979, sialylated oligosaccharides can be made as described in WO 2012/113404 and mixtures of human milk oligosaccharides can be made as described in WO 2012/113405. As examples of enzymatic production, sialylated oligosaccharides can be made as described in WO 2012/007588, fucosylated oligosaccharides can be made as described in WO 2012/127410, and advantageously diversified blends of human milk oligosaccharides can be made as described in WO 2012/156897 and WO 2012/156898. With regard to biotechnological methods, WO 01/04341 and WO 2007/101862 describe how to make core human milk oligosaccharides optionally substituted by fucose or sialic acid using genetically modified *E. coli.*

The synthetic composition of this invention comprising one or human milk monosaccharides or one or more human milk oligosaccharides, or both can take any suitable form. For example, the composition can be in the form of a nutritional composition which contains other macronutrients such as proteins, lipids or other carbohydrates. The synthetic composition can also be a pharmaceutical composition. In one embodiment, the synthetic compositions contains one or more backbone HMOs and one or more fucosyl HMOs. In another embodiment, the synthetic composition contains one or more backbone HMOs and one or more sialyl HMOs. In a further embodiment, the synthetic composition comprises one or more fucosyl HMOs and one or more sialyl HMOs. In a preferred embodiment, the synthetic composition contains one or more backbone HMOs, one or more sialyl HMOs and one or more fucosyl HMOs.

### Nutritional compositions

A nutritional composition of this invention can contain sources of protein, lipids and/or digestible carbohydrates and can be in powdered or liquid forms. The composition can be designed to be the sole source of nutrition or a nutritional supplement.

Suitable protein sources include milk proteins, soy protein, rice protein, pea protein and oat protein, or mixtures thereof. Milk proteins can be in the form of milk protein concentrates, milk protein isolates, whey protein or casein, or mixtures of both. The protein can be whole protein or hydrolysed protein, either partially hydrolysed or extensively hydrolysed. Hydrolysed protein offers the advantage of easier digestion which can be important for patients with inflamed GI tracts. The protein can also be provided in the form of free amino acids. The protein can comprise about 5 % to about 30 % of the energy of the nutritional composition, normally about 10 % to 20 %.

The protein source can be a source of glutamine, threonine, cysteine, serine, proline, or a combination of these amino acids. The glutamine source can be a glutamine dipeptide and/or a glutamine enriched protein. Glutamine can be included due to the use of glutamine by enterocytes as an energy source. Threonine, serine and proline are important amino acids for the production of mucin. Mucin coats the GI tract and can improve mucosal healing. Cysteine is a major precursor of glutathione, which is key for the antioxidant defences of the body.

Suitable digestible carbohydrates include maltodextrin, hydrolysed or modified starch or corn starch, glucose polymers, corn syrup, corn syrup solids, high fructose corn syrup, rice-derived carbohydrates, pea-derived carbohydrates, potato-derived carbohydrates, tapioca, sucrose, glucose, fructose, sucrose, lactose, honey, sugar alcohols (e.g., maltitol, erythritol, sorbitol), or mixtures thereof. Preferably the composition is free from lactose. Generally digestible carbohydrates provide about 35 % to about 55 % of the energy of the nutritional composition. Preferably the nutritional composition is free from lactose. A particularly suitable digestible carbohydrate is a low dextrose equivalent (DE) maltodextrin.

Suitable lipids include medium chain triglycerides (MCT) and long chain triglycerides (LCT). Preferably the lipid is a mixture of MCTs and LCTs. For example, MCTs can comprise about 30 % to about 70 % by weight of the lipids, more specifically about 50 % to about 60 % by weight. MCTs offer the advantage of easier digestion which can be important for patients with inflamed GI tracts. Generally the lipids provide about 35 % to about 50 % of the energy of the nutritional composition. The lipids can contain essential fatty acids (omega-3 and omega-6 fatty acids). Preferably these polyunsaturated fatty acids provide less than about 30 % of total energy of the lipid source. Decreasing the levels of these polyunsaturated fatty acids is believed to decrease sensitivity to peroxidation; which can be beneficial for patients having inflammatory conditions.

Suitable sources of long chain triglycerides are rapeseed oil, sunflower seed oil, palm oil, soy oil, milk fat, corn oil, high oleic oils, and soy lecithin. Fractionated coconut oils are a suitable source of medium chain triglycerides. The lipid profile of the nutritional composition is preferably designed to have a polyunsaturated fatty acid omega-6 (n-6) to omega-3 (n-3) ratio of about 4:1 to about 10:1. For example, the n-6 to n-3 fatty acid ratio can be about 6:1 to about 9:1.

The nutritional composition preferably also includes vitamins and minerals. If the nutritional composition is intended to be a sole source of nutrition, it preferably includes a complete vitamin and mineral profile. Examples of vitamins include vitamins A, B-complex (such as B1, B2, B6 and B12), C, D, E and K, niacin and acid vitamins such as pantothenic acid, folic acid and biotin. Examples of minerals include calcium, iron, zinc, magnesium, iodine, copper, phosphorus, manganese, potassium, chromium, molybdenum, selenium, nickel, tin, silicon, vanadium and boron.

The nutritional composition can also include a carotenoid such as lutein, lycopene, zeaxanthin, and beta-carotene. The total amount of carotenoid included can vary from about 0.001 µg/ml to about 10 µg/ml. Lutein can be included in an amount of from about 0.001 µg/ml to about 10 µg/ml, preferably from about 0.044 µg/ml to about 5 g/ml of lutein. Lycopene can be included in an amount from about 0.001 µg/ml to about 10 µg/ml, preferably about 0.0185 mg/ml to about 5 g/ml of lycopene. Beta-carotene can comprise from about 0.001 µg/ml to about 10 mg/ml, for example about 0.034 µg/ml to about 5 µg/ml of beta-carotene.

The nutritional composition preferably also contains reduced concentrations of sodium; for example from about 300 mg/l to about 400 mg/l. The remaining electrolytes can be present in concentrations set to meet needs without providing an undue renal solute burden on kidney function. For example, potassium is preferably present in a range of about 1180 to about 1300 mg/l; and chloride is preferably present in a range of about 680 to about 800 mg/l.

The nutritional composition can also contain various other conventional ingredients such as preservatives, emulsifying agents, thickening agents, buffers, fibres and prebiotics (e.g. fructooligosaccharides, galactooligosaccharides), probiotics (e.g. *B. animalis* subsp. lactis BB-12, *B*. *lactis* HN019, *B. lactis* Bi07, *B. infantis* ATCC 15697, *L. rhamnosus* GG, *L. rhamnosus* HNOOI, *L. acidophilus* LA-5, *L. acidophilus* NCFM, *L. fermentum* CECT5716, *B. longum* BB536, *B. longum* AH1205, *B. longum* AH1206, *B. breve* M-16V, *L. reuteri* ATCC 55730, *L. reuteri* ATCC PTA-6485, *L. reuteri* DSM 17938), antioxidant/anti-inflammatory compounds including tocopherols, caroteinoids, ascorbate/vitamin C, ascorbyl palmitate, polyphenols, glutathione, and superoxide dismutase (melon), other bioactive factors (e.g. growth hormones, cytokines, TFG-β), colorants, flavours, and stabilisers, lubricants, and so forth.

The nutritional composition can be in the form of a soluble powder, a liquid concentrate, or a ready-to-use formulation. The composition can be fed to a patient via a nasogastric tube or by having the patient drink it. Various flavours, fibres and other additives can also be present.

The nutritional compositions can be prepared by any commonly used manufacturing techniques for preparing nutritional compositions in solid or liquid form. For example, the composition can be prepared by combining various feed solutions. A protein-in-fat feed solution can be prepared by heating and mixing the lipid source and then adding an emulsifier (e.g. lecithin), fat soluble vitamins, and at least a portion of the protein source while heating and stirring. A carbohydrate feed solution is then prepared by adding minerals, trace and ultra trace minerals, thickening or suspending agents to water while heating and stirring. The resulting solution is held for 10 minutes with continued heat and agitation before adding carbohydrates (e.g. the HMOs and digestible carbohydrate sources). The resulting feed solutions are then blended together while heating and agitating and the pH adjusted to 6.6-7.0, after which the composition is subjected to high-temperature short-time processing during which the composition is heat treated, emulsified and homogenized, and then allowed to cool. Water soluble vitamins and ascorbic acid are added, the pH is adjusted to the desired range if necessary, flavours are added, and water is added to achieve the desired total solid level.

For a liquid product, the resulting solution can then be aseptically packed to form an aseptically packaged nutritional composition. In this form, the nutritional composition can be in ready-to-feed or concentrated liquid form. Alternatively the composition can be spray-dried and processed and packaged as a reconstitutable powder.

When the nutritional product is a ready-to-feed nutritional liquid, the total concentration of HMSs/HMOs in the liquid, by weight of the liquid, is from about 0.0001 % to about 2.0 %, including from about 0.001 % to about 1.5 %, including from about 0.01 % to about 1.0 %. When the nutritional product is a concentrated nutritional liquid, the total concentration of HMSs/HMOs in the liquid, by weight of the liquid, is from about 0.0002 % to about 4.0 %, including from about 0.002 % to about 3.0 %, including from about 0.02 % to about 2.0 %.

### Unit dosage forms

The synthetic composition of this invention can also be in a unit dosage form such as a capsule, tablet or sachet. For example, the composition can be in a tablet form comprising the human milk monosaccharides and/or oligosaccharides, and one or more additional components to aid formulation and administration, such as diluents, excipients, antioxidants, lubricants, colorants, binders, disintegrants, and the like.

Suitable diluents, excipients, lubricants, colorants, binders, and disintegrants include polyethylene, polyvinyl chloride, ethyl cellulose, acrylate polymers and their copolymers, hydroxyethyl-cellulose, hydroxypropylmethyl-cellulose (HPMC), sodium carboxymethylcellulose, polyhydroxyethyl methacrylate (PHEMA), polyvinyl alcohol (PVA), polyvinyl pyrrolidone (PVP), polyethylene oxide (PEO), or polyacrylamide (PA), carrageenan, sodium alginate, polycarbophil, polyacrylic acid, tragacanth, methyl cellulose, pectin, natural gums, xanthan gum, guar gum, karaya gum, hypromellose, magnesium stearate, microcrystalline cellulose, and colloidal silicon dioxide. Suitable antioxidants are vitamin A, carotenoids, vitamin C, vitamin E, selenium, flavonoids, polyphenols, lycopene, lutein, lignan, coenzyme Q10 ("CoQIO") and glutathione.

The unit dosage forms, especially those in sachet form, can also include various nutrients including macronutrients.

### Administration dosing

For inducing and/or promoting mucosal healing in a patient, preferably a human, the amount of human milk mono and/or oligosaccharide(s) required to be administered to the patient will vary depending upon factors such as the risk and severity of an inflammatory gastrointestinal condition or an IBD, the age of the patient, the form of the composition, and other medications being administered to the patient. However, the required amount can be readily set by a medical practitioner and would generally be in the range from about 10 mg to about 20 g per day, in certain embodiments from about 10 mg to about 15 g per day, from about 100 mg to about 10 g per day, from about 500 mg to about 10 g per day, or from about 1 g to about 7.5 g per day, and in other embodiments from about 50 mg to about 20 g per day, from about 100 mg to about 20 g per day, from about 500 mg to about 15 g per day, or from about 1 g to about 10 g per day. An appropriate dose can be determined based on several factors, including, for example, the body weight and/or condition of the patient being treated, the severity of the inflammatory gastrointestinal condition or IBD, being treated, other ailments and/or diseases of the patient, the incidence and/or severity of side effects and the manner of administration. Appropriate dose ranges may be determined by methods known to those skilled in the art. During an initial treatment phase, the dosing can be higher (for example 200 mg to 20 g per day, in certain embodiments preferably 500 mg to 15 g per day, more preferably 1 g to 10 g per day, even more preferably 2.5 g to 7.5 g per day, and in other embodiments preferably 500 mg to 20 g per day, more preferably 1 g to 20 g per day, even more preferably 1.5 g to 2.5 g per day). During a maintenance phase, the dosing can be reduced (for example, to 10 mg to 10 g per day, in certain embodiments preferably to 100 mg to 7.5 g per day, more preferably to 500 mg to 5 g per day, even more preferably to 1 g to 2.5 g per day, and in other embodiments preferably to 100 mg to 10 g per day, more preferably to 500 mg to 7.5 g per day, even more preferably to 1 g to 5 g per day).

A synthetic composition of this invention can be co-administered to a patient who is also receiving a standard-of-care medication such as a 5-aminosalicylate, a corticosteroid or anti-TNF-a treatment to improve the treatment protocol by promoting mucosal healing. This can more rapidly move the patient into remission, allowing dosing of the standard-of-care medication to be reduced. Similarly, the synthetic composition of this invention can maintain the patient in remission for longer through maintenance of mucosal integrity.

### EXAMPLES

Examples are now described to further illustrate the invention:

### Example 1 - Treatment of acute inflammation model

A dextran sulphate sodium (DSS) mouse model is used. DSS interferes with intestinal barrier function and then stimulates local inflammation. Therefore DSS induces a form of mouse colitis that mimics the clinical and histological features of IBDs that have characteristics of UC. Two hundred and forty male C57BL/6 mice weighing about 20 - 25 g are housed in individual cages at controlled temperature (22 °C) with a 12 hour light: dark cycle. They are divided into 12 groups of 20 mice; a control group and 11 treatment groups. All mice are fed the same dry rodent chow diet except that the diet of the control mice includes cellulose (50 g/kg of diet) and each treatment group includes cellulose (30 g/kg of diet) and an HMS/HMO (20 g/kg of diet). The HMS/HMO in the treatment groups are sialic acid, L-fucose, 2'-FL, 3-FL, 3'-SL, 6'-SL, LNT, LNnT, LNFP-I, DSLNT, a combination of each of these saccharides. Fresh food is given daily. All mice have free access to drinking water.

After a 7 day adaptation period, the control and treatment mice are each randomly distributed into two experimental groups. One group, called the DSS group, is treated with DSS dissolved in drinking water. DSS concentration is about 2-4 % for the first 4 days and the mice are then moved over to a recovery period where no DSS is administered. The other group of mice, called the pair-fed (PF) group, are not given DSS and is fed the mean amount of food consumed by the DSS group.

During the treatment, body weight, food intake, and the presence of blood in the stools, is assessed daily. Direct visualization of colonic mucosal damage and healing in vivo is performed using the Coloview (Karl Storz Veterinary Endoscopy, Tuttlingen, Germany). Mice are anesthetized with 1.5 to 2 % isoflurane and 3 cm of the colon proximal to the anus is visualized after inflation of the colon with air. Mucosal healing is assessed visually using four descriptors
- vascular pattern (normal - 0, partially visible - 1, complete loss - 2)
- granularity (normal - 0, fine - 1, course - 2)
- ulceration (normal - 0, erosions - 1, shallow - 2, deep - 3, diffuse > 30 % deep - 4)
- bleeding/friability (normal - 0, friable - 1, spontaneous bleeding - 2).

The score is 3.1 x vascular pattern + 3.6 x granularity + 3.5 x ulceration + 2.5 x bleeding/friability.

At the end of the protocol, the mice are anesthetised with sodium pentobarbital. The mice are exsanguinated by sampling blood from abdominal aorta and plasma is separated by centrifugation and kept at -80 °C until analysis. The following tissue is quickly removed, blotted dry, weighed, frozen in liquid nitrogen and stored at -80 °C until analysis: spleen, thymus, liver, lung and gastrocnemius and soleus muscles. The ileum and caecum are removed, rinsed with cold PBS, weighed and stored at -80 °C until analysis. The colon is flushed with cold PBS, dried, weighed and divided into sections for mucosal extraction and histological evaluation and the rest is stored at - 80 °C until analysis. All procedures are performed according to current legislation on animal experiment in Europe.

The presence of diarrhoea and/or blood in the stools is evaluated following a scoring system: 0 = no blood, no diarrhoea; 0.5 = no blood with diarrhoea; 1 = little blood with diarrhoea; 1.5 = no blood with severe diarrhoea; 2 = little blood with severe diarrhoea; 2.5 = extensive blood with severe diarrhoea. Blood cell counts (white and red cells, platelet and haematocrit) are determined with an automatic haematology counter on fresh blood. Colonic full-thickness (5 mm) sections are stained with haematoxylin and eosin and analysed by two pathologists blinded to the experimental groups. Microphotographs are taken with a Leica DM 500B microscope.

Caecum and colon are assayed for myeloperoxidase (MPO) activity. MPO activity is widely accepted as a marker of the infiltration of neutrophils into tissues, and is correlated with white cells counts and associated with colon histological lesions in DSS-treated mice.

Gene expression, including for cytokines, is determined as follows. Total RNA is extracted using TRIzol reagent (Invitrogen, Carlsbad, CA) and reverse-transcribed using the cDNA Synthesis kit (Fermentas, Glen Burnie, MD). RT-PCR is performed using the GeneJET Fast PCR kit (Fermentas, Glen Burnie, MD). Real-time RT-PCR is performed using an iCycler (Bio-Rad, Hercules, CA). Briefly, cDNA is amplified by 40 cycles of 95 °C for 15 s and 60 °C for 1 minute, using the iQ SYBR Green Supermix system (Biorad, Hercules, CA) and specific primers. During the 7 days prior to DDS treatment, body weight gain and food intake are similar in the control and treatment mice. In DSS-control mice, food intake is reduced during the treatment and the recovery period. In DSS-treatment mice, food intake decreases initially but then recovers. Body weight follows the same pattern. The gastrocnemius, liver, thymus and ileum weight of DSS-treatment mice is lower than PF-control but higher than DSS-control.

Similar results are obtained for colon weight/length ratio, indicating a weaker local inflammatory response. DSS-control mice have a higher average stool score than DSS-treatment mice.

DSS treatment causes an increase in leukocytes in both control and treatment mice; especially granulocytes. The DDS-treatment group have less leukocyte increase than the DSS-control group.

Colon MPO activity is higher in DSS-control mice compared to their PF control. In contrast, colon M PO activity in DSS-treatment mice does not statistically differ from that of their PF group.

Gene expression analysis reveals that DSS treatment causes a reduction in the expression of growth factors relevant to intestinal maturation and repair. However the mice in the DSS-treatment group have increased expression of the gene for epidermal growth factor related protein, up to normal values. Recuperation of expression of the gene for placenta growth factor, which is involved in angiogenesis regulation, is evident in the DSS-treatment mice. A group of genes encoding mucins (MUC-1, MUC-2, MUC-3, MUC-5) are down-regulated by DSS treatment but their expression tend to be normal in DSS-treatment mice; especially MUC-2. Alkaline phosphatase, a marker of IBD, is induced by DSS treatment but its expression returns to normal in DSS-treatment mice. The genes involved in inflammation, namely IL-1b, are strongly induced by DSS treatment. However the DSS-treatment mice have decreased expression of this and other inflammatory cytokines compared to DSS-control mice. IL-10 expression is upregulated in DSS-treatment mice as compared DSS-control mice.

The HMS/HMO protect the mice from the toxicity of DSS since mice fed the HMS/HMO show less severe clinical symptoms (diarrhoea, colonic damage and bloody stools) than DSS-control mice. Further the treatment mice have a better recovery from the acute inflammatory process, as shown by less severe colonic lesions and clinical symptoms. The treatment mice also showed a weaker inflammatory response. The DSS-treatment mice have improved mucosal healing as compared to the DSS-control mice.

### Example 2 - Treatment of chronic inflammation model

IL-10 knock out mice are used because they develop a chronic condition close to human CD. IL-10 knock-out mice are housed and bred in specific pathogen free conditions. At four weeks after birth, the mice are exposed to an environmental trigger to induce colitis (in this case housing under non-SPF conditions). Under these conditions, close to 100% of the mice are expected to develop moderate to severe colitis. The mice are separated into twelve groups of 10 mice as follows:
Group 1- control
Group 2 - sialic acid
Group 3 - L-fucose
Group 4 - 2'-FL
Group 5 - 3-FL
Group 6 - 3'-SL
Group 7 - 6'-SL
Group 8 - LNT
Group 9 - LNnT
Group 10 - LNFP-I
Group 11 - DSLNT
Group 12 - a combination of each of the saccharides above.

For the treatment groups (2 - 12), the saccharide treatment is commenced upon weaning. The saccharides are added to the drinking water at a concentration of 5 mM. Fresh water is administered daily and all mice have free access to the drinking water. The mice are fed a rodent chow and are given fresh chow daily. The mice are fed the saccharides for a duration of 4 weeks.

The mice are assessed weekly on each of the following parameters: excreted perianal mucus, rectal prolapse, mucosal damage and healing by endoscopy, diarrhoea and weight loss. Scores of 0 or 1 are given to obtain a clinical score which resembles the Crohn's Disease Activity Index. Stool samples are collected weekly. The samples are subjected to microbiota analysis (16S RNA pyrosequencing) and are emulsified in 500 µl/100 µg stool in PBS containing soy trypsin inhibitor and phenyl-methylsulphonyl fluoride and centrifuged. The supernatants are collected and kept frozen at -20 °C until use. Gene expression is measured as described in Example 1.

Mucosal healing is visually assessed using a modified Crohn's Disease Endoscopic Index of Severity (CDEIS) scoring system.

Clinical parameters and biomarker results are similar to those obtained in Example 1 with the treatment mice having reduced levels of pro-inflammatory cytokines, increase expression of MUC-2 and improved mucosal healing scores.

### Example 3 - Preventative treatment of chronic inflammation model

The protocol of example 2 is repeated except that (1) there are only two groups of mice, the control group and a treatment group receiving a combination of all HMS/HMO; and (2) the intervention commences 1 week prior to exposure to the environmental trigger.

The treatment mice have delayed/limited development of colitis as compared to the control group. This indicates the preventative properties of the HMS/HMO. Other parameters are similar to those obtained in example 2.

### Example 4 - Treatment of chronic condition close to human inflammation model

MUC2 knock out mice are used because they develop a chronic condition close to human UC. MUC2 knock-out mice are housed and bred in specific pathogen free conditions. At four weeks after birth, the mice are exposed to an environmental trigger to induce colitis (in this case housing under non-SPF conditions). Under these conditions, close to 100% of the mice are expected to develop moderate to severe colitis. The mice are separated into twelve groups of 10 mice as follows:
Group 1- control
Group 2 - sialic acid
Group 3 - L-fucose
Group 4 - 2'-FL
Group 5 - 3-FL
Group 6 - 3'-SL
Group 7 - 6'-SL
Group 8 - LNT
Group 9 - LNnT
Group 10 - LNFP-I
Group 11 - DSLNT
Group 12 - a combination of each of the saccharides above.

For the treatment groups (2 - 12), the saccharide treatment is commenced upon weaning. The saccharides are added to the drinking water at a concentration of 5 mM. Fresh water is administered daily and all mice have free access to the drinking water. The mice are fed a rodent chow and are given fresh chow daily. The mice are fed the saccharides for a duration of 4 weeks.

The mice are assessed weekly on each of the following parameters: excreted perianal mucus, rectal prolapse, mucosal damage and healing by endoscopy, diarrhoea and weight loss. Scores of 0 or 1 are given to obtain a clinical score which resembles the Crohn's Disease Activity Index. Stool samples are collected weekly. The samples are subjected to microbiota analysis (16S RNA pyrosequencing) and are emulsified in 500 µl/100 µg stool in PBS containing soy trypsin inhibitor and phenyl-methylsulphonyl fluoride and centrifuged. The supernatants are collected and kept frozen at -20 °C until use. Gene expression is measured as described in Example 1.

Mucosal healing is visually assessed using a modified Crohn's Disease Endoscopic Index of Severity (CDEIS) scoring system.

Clinical parameters and biomarker results are similar to those obtained in Example 1 with the treatment mice having reduced levels of pro-inflammatory cytokines and improved mucosal healing scores.

### Example 5 - Preventative treatment of chronic condition close to human inflammation model

The protocol of example 4 is repeated except that (1) there are only two groups of mice, the control group and a treatment group receiving a combination of all HMS/HMO; and (2) the intervention commences 1 week prior to exposure to the environmental trigger.

The treatment mice have delayed/limited development of colitis as compared to the control group. This indicates the preventative properties of the HMS/HMO. Other parameters are similar to those obtained in example 4.

### Example 6 - Mucosal barrier function model

Sialic acid, L-fucose, 2'-FL, 3-FL, 3'-SL, 6'-SL, LNT, LNnT, LNFP-I, DSLNT and a combination of each of these saccharides are tested with respect to their ability to induce MUC2, TFF3, EIMβ, CHST5, and GAL3ST2 expression in the human LS174T cell culture model of goblet cells. LS174T cells (ATCC) are maintained in minimum essential medium (MEM) supplemented according to instructions at 37 °C in 5 % CO₂. Sialic acid, L-fucose, 2'-FL, 3-FL, 3'-SL, 6'-SL, LNT, LNnT, LNFP-I, DSLNT and a combination of each of these saccharides are dissolved in cell culture grade water to the required concentration. The LS174T cells are treated with the HMS/HMO solution containing 0 or 5 mg HMS/HMO per ml.

The LS174T cells are collected and suspended in Trizol reagent and total RNA is isolated using an RNA analysis kit (Qiagen) according to the manufacturer's instructions and the RNA isolates are quantified using Nanodrop analysis (Thermo Fisher Scientific). RNA isolates are reverse transcribed using a high capacity cDNA Reverse Transcription Kit (Applied Biosystems) to create cDNA, which is then used to assess gene expression via quantitative PCR.

For the quantitative RT-PCR, specific TaqMAN gene expression assays are obtained from Applied Biosystems, which include expression assays for MUC2, TFF3, CHST5 and GAL3ST2. Quantitative real-time PCR is performed using TaqMAN PCR Master Mix (Applied Biosystems). Reactions are run in duplicates in a 384-well plate using an Applied Biosystems 7900HT Fast Real-Time PCR System. The results are analysed using SDS 2.3 software and calculated by delta Ct method. All samples are normalized to Gus-β expression and fold induction is calculated over untreated controls. Gene expression is expressed as fold increase compared to HMS/HMO-free control cells. The experiment is repeated three times.

The results indicate that the HMSs/HMOs and their combination increase the expression of the MUC2 and TFF3 genes compared to control cultures. Increased expression of goblet cell genes is specific and not universal, as evidenced by the minimal induction or lack of induction of CHST5 and GAL3ST2, respectively. MUC2 and TFF3 are key components of the mucosal barrier and improve mucosal barrier function.

### Example 7 - Nutritional composition

A ready to feed nutritional composition is prepared from water, maltodextrin, enzymatically hydrolysed whey protein (from cows milk), medium chain triglycerides (from coconut and/or palm kernel oil), cornstarch, soybean oil, soy lecithin, HMSs/HMOs, magnesium chloride, calcium phosphate, guar gum, sodium ascorbate, potassium citrate, sodium phosphate, calcium citrate, choline chloride, potassium chloride, sodium citrate, magnesium oxide, taurine, L-carnitine, alpha-tocopheryl acetate, zinc sulphate, ferrous sulphate, niacinamide, calcium pantothenate, vitamin A palmitate, citric acid, manganese sulphate, pyridoxine hydrochloride, vitamin D3, copper sulphate, thiamine mononitrate, riboflavin, beta carotene, folic acid, biotin, potassium iodide, chromium chloride, sodium selenate, sodium molybdate, phytonadione, vitamin B12.

The composition has a calorific density of 1.0 kcal/ml with a caloric distribution (% of kcal) as follows: protein: 16 %, carbohydrate: 51 %, fat: 33 %. The protein source has an NPC:N ratio of 131:1. The MCT:LCT ratio is 70:30 and the n6:n3 ratio is 7.4:1. The osmolality (mOsm/kg water) is 270 when unflavoured. The composition contains 85 % water and 1500 ml meets 100 % of the RDI for 22 key micronutrients.

The composition has a balanced peptide profile which promotes GI absorption and integrity and the enzymatically hydrolysed 100 % whey protein facilitates gastric emptying. The MCT decreases the potential for fat malabsorption. The composition is nutritionally complete for tube feeding or oral supplementation.

### Example 8 - Tablet composition

A tablet is prepared from HMS/HMO, hydroxypropyl methylcellulose, sodium alginate, gum, microcrystalline cellulose, colloidal silicon dioxide, and magnesium stearate. All raw materials except the magnesium stearate are placed into a high shear granulator and premixed. Water is sprayed onto the premix while continuing to mix at 300 rpm. The granulate is transferred to a fluidised bed drier and dried at 75 °C. The dried powder is sieved and sized using a mill. The resulting powder is then lubricated with magnesium stearate and pressed into tablets. The tablets each contain 325 mg of HMS/HMO. The tablets each have a weight of 750 mg.

### Example 9 - Capsule composition

A capsule is prepared by filling about 1 g of HMS/HMO into a 000 gelatine capsule using a filing machine. The capsules are then closed. The HMS/HMO are in free flowing, powder form.

## Claims

1. One or more human milk oligosaccharides (HMOs) for use in:
- treating a patient with IBD having ulceration to induce remission by inducing and/or promoting mucosal healing in the patient,
- maintaining a patient with IBD in remission by inducing/promoting mucosal healing in the patient and maintaining mucosal integrity,
- promoting mucosal healing in a patient having ulceration due to an inflammatory gastrointestinal condition, and/or
- inducing mucosal healing in a patient having ulceration due to an inflammatory gastrointestinal condition.

2. A synthetic composition for use in:
- treating a patient with IBD having ulceration to induce remission by inducing and/or promoting mucosal healing in the patient,
- maintaining a patient with IBD in remission by inducing/promoting mucosal healing in the patient and maintaining mucosal integrity,
- promoting mucosal healing in a patient having ulceration due to an inflammatory gastrointestinal condition, and/or
- inducing mucosal healing in a patient having ulceration due to an inflammatory gastrointestinal condition,
**characterised in that** the composition contains an effective amount of one or more human milk oligosaccharides (HMOs).

3. The one or more HMOs for the use according to claim 1 or the synthetic composition for the use according to claim 2, wherein the patient is human.

4. The one or more HMOs for the use according to claim 1 or 3, or the synthetic composition for the use according to claim 2 or 3, wherein the use is:
- treating patient with IBD having ulceration to induce remission by inducing and/or promoting mucosal healing in the patient, and/or
- maintaining a patient with IBD in remission by inducing/promoting mucosal healing in the patient and maintaining mucosal integrity.

5. The one or more HMOs or the synthetic composition for the use according to claim 4, wherein the patient with IBD is a patient with moderate-to-severe IBD who is receiving immunomodulator treatment.

6. The one or more HMOs for the use according to claim 1 or 3, or the synthetic composition for the use according to claim 2 or 3, wherein the use is promoting mucosal healing in a patient having ulceration due to an inflammatory gastrointestinal condition.

7. The one or more HMOs for the use according to claim 1 or 3, or the synthetic composition for the use according to claim 2 or 3, wherein the use is inducing mucosal healing in a patient having ulceration due to an inflammatory gastrointestinal condition.

8. The one or more HMOs for the use according to any of claims 1 or 3 to 7, or the synthetic composition for the use according to any of claims 2 or 7, wherein patient is a paediatric or adult patient.

9. The synthetic composition for the use according to any of claims 2 to 8, which is a nutritional composition.

10. The synthetic composition for the use according to any of claims 2 to 9, further comprising a source of threonine, serine and/or proline.

11. The one or more HMOs for the use according to any of claims 1 or 3 to 8, or the synthetic composition for the use according to any of claims 2 to 10, wherein the HMOs comprise
- one or more backbone HMOs and one or more fucosyl HMOs,
- one or more backbone HMOs and one or more sialyl HMOs, or
- one or more fucosyl HMOs and one or more sialyl HMOs.

12. The one or more HMOs for the use according to any of claims 1 or 3 to 8, or the synthetic composition for the use according to any of claims 2 to 10, wherein the HMOs comprise one or more backbone HMOs, one or more sialyl HMOs and one or more fucosyl HMOs.

13. The one or more human milk oligosaccharides (HMOs) for the use according to any of claims 1 or 3 to 8, or the synthetic composition for the use according to any of claims 2 to 10, wherein the human milk oligosaccharide is selected from 2'-FL, 3-FL, DFL, 3'-SL, 6'-SL, LNT, LNnT, LNFP-I and DSLNT.

## Patentansprüche

1. Ein oder mehrere humane Milch-Oligosaccharide (human milk oligosaccharides, HMOs) zur Anwendung bei:
- Behandeln eines Patienten mit IBD, der Ulzeration aufweist, um durch Herbeiführen und/oder Fördern von mukosaler Heilung eine Remission zu veranlassen,
- Beibehalten eines Patienten mit IBD in Remission durch Herbeiführen/Fördern von mukosaler Heilung im Patienten und Aufrechterhalten mukosaler Integrität,
- Fördern mukosaler Heilung in einem Patienten, der infolge einer entzündlichen gastrointestinalen Erkrankung Ulzeration aufweist, und/oder
- Herbeiführen mukosaler Heilung in einem Patienten, der infolge einer entzündlichen gastrointestinalen Erkrankung Ulzeration aufweist.

2. Eine synthetische Zusammensetzung zur Anwendung bei:
- Behandeln eines Patienten mit IBD, der Ulzeration aufweist, um durch Herbeiführen und/oder Fördern von mukosaler Heilung im Patienten Remission zu veranlassen,
- Beibehalten eines Patienten mit IBD in Remission durch Herbeiführen/Fördern von mukosaler Heilung im Patienten und Aufrechterhalten mukosaler Integrität,
- Fördern mukosaler Heilung in einem Patienten, der infolge einer entzündlichen gastrointestinaler Erkrankung Ulzeration aufweist, und/oder
- Herbeiführen mukosaler Heilung in einem Patienten, der infolge einer entzündlichen gastrointestinalen Erkrankung Ulzeration aufweist,
**dadurch gekennzeichnet, dass** die Zusammensetzung eine wirksame Menge von einem oder mehreren humanen Milch-Oligosacchariden (HMOs) enthält.

3. Ein oder mehrere HMOs zur Anwendung nach Anspruch 1 oder die synthetische Zusammensetzung zur Anwendung nach Anspruch 2, wobei der Patient ein Mensch ist.

4. Ein oder mehrere HMOs zur Anwendung nach Anspruch 1 oder 3 oder die synthetische Zusammensetzung zur Anwendung nach Anspruch 2 oder 3, wobei die Anwendung folgende ist:
- Behandeln eines Patienten mit IBD, der Ulzeration aufweist, um Remission durch Herbeiführen und/oder Fördern mukosaler Heilung im Patienten zu veranlassen, und/oder
- Beibehalten eines Patienten mit IBD in Remission durch Herbeiführen/Fördern mukosaler Heilung im Patienten und Aufrechterhalten mukosaler Integrität.

5. Ein oder mehrere HMOs oder die synthetische Zusammensetzung zur Anwendung nach Anspruch 4, wobei der Patient mit IBD ein Patient mit mittelschwerer bis schwerer IBD ist, der eine Immunomodulatorbehandlung erhält.

6. Ein oder mehrere HMOs zur Anwendung nach Anspruch 1 oder 3 oder die synthetische Zusammensetzung zur Anwendung nach Anspruch 2 oder 3, wobei die Anwendung mukosale Heilung in einem Patienten fördert, der infolge einer entzündlichen gastrointestinalen Erkrankung Ulzeration aufweist.

7. Ein oder mehrere HMOs zur Anwendung nach Anspruch 1 oder 3 oder die synthetische Zusammensetzung zur Anwendung nach Anspruch 2 oder 3, wobei die Anwendung mukosale Heilung in einem Patienten herbeiführt, der infolge einer entzündlichen gastrointestinalen Erkrankung Ulzeration aufweist.

8. Ein oder mehrere HMOs zur Anwendung nach einem der Ansprüche 1 oder 3 bis 7 oder die synthetische Zusammensetzung zur Anwendung nach einem der Ansprüche 2 oder 7, wobei der Patient ein pädiatrischer oder erwachsener Patient ist.

9. Die synthetische Zusammensetzung zur Anwendung nach einem der Ansprüche 2 bis 8, die eine Nährstoffzusammensetzung ist.

10. Die synthetische Zusammensetzung zur Anwendung nach einem der Ansprüche 2 bis 9, ferner umfassend eine Quelle von Threonin, Serin und/oder Prolin.

11. Ein oder mehrere HMOs zur Anwendung nach einem der Ansprüche 1 oder 3 bis 8 oder die synthetische Zusammensetzung zur Anwendung nach einem der Ansprüche 2 bis 10, wobei die HMOs Folgendes umfassen:
- ein oder mehrere Rückgrat-HMOs und ein oder mehrere Fucosyl-HMOs,
- ein oder mehrere Rückgrat-HMOs und ein oder mehrere Sialyl-HMOs oder
- ein oder mehrere Fucosyl-HMOs und ein oder mehrere Sialyl-HMOs.

12. Ein oder mehrere HMOs zur Anwendung nach einem der Ansprüche 1 oder 3 bis 8 oder die synthetische Zusammensetzung zur Anwendung nach einem der Ansprüche 2 bis 10, wobei die HMOs ein oder mehrere Rückgrat-HMOs, ein oder mehrere Sialyl-HMOs und ein oder mehrere Fucosyl-HMOs umfassen.

13. Ein oder mehrere humane Milch-Oligosaccharide (HMOs) zur Anwendung nach einem der Ansprüche 1 oder 3 bis 8 oder die synthetische Zusammensetzung zur Anwendung nach einem der Ansprüche 2 bis 10, wobei das humane Milch-Oligosaccharid aus 2'-FL, 3-FL, DFL, 3'-SL, 6'-SL, LNT, LNnT, LNFP-I und DSLNT ausgewählt wird.

## Revendications

1. Un ou plusieurs oligosaccharides du lait humain (HMO) pour leur utilisation dans :
- le traitement d'un patient avec une maladie intestinale inflammatoire (MII) ayant une ulcération pour induire la rémission par induction et/ou favorisation de la cicatrisation des muqueuses chez le patient,
- le maintien d'un patient avec une MII en rémission par induction/favorisation de la cicatrisation des muqueuses chez le patient et maintien de l'intégrité des muqueuses,
- la favorisation de la cicatrisation des muqueuses chez un patient ayant une ulcération due à une affection gastro-intestinale inflammatoire, et/ou
- l'induction de la cicatrisation des muqueuses chez un patient ayant une ulcération due à une affection gastro-intestinale inflammatoire.

2. Une composition synthétique pour son utilisation dans :
- le traitement d'un patient avec une MII ayant une ulcération pour induire la rémission par induction et/ou favorisation de la cicatrisation des muqueuses chez le patient,
- le maintien d'un patient avec une MII en rémission par induction/favorisation de la cicatrisation des muqueuses chez le patient et maintien de l'intégrité des muqueuses,
- la favorisation de la cicatrisation des muqueuses chez un patient ayant une ulcération due à une affection gastro-intestinale inflammatoire, et/ou
- l'induction de la cicatrisation des muqueuses chez un patient ayant une ulcération due à une affection gastro-intestinale inflammatoire,
**caractérisés en ce que** la composition contient une quantité efficace d'un ou plusieurs oligosaccharides du lait humain (HMO).

3. Un ou plusieurs HMO pour leur utilisation selon la revendication 1 ou la composition synthétique pour son utilisation selon la revendication 2, dans lesquels le patient est humain.

4. Un ou plusieurs HMO pour leur utilisation selon la revendication 1 ou 3, ou la composition synthétique pour son utilisation selon la revendication 2 ou 3, dans lesquels l'utilisation est :
- le traitement d'un patient avec une MII ayant une ulcération pour induire la rémission par induction et/ou favorisation de la cicatrisation des muqueuses chez le patient, et/ou
- le maintien d'un patient avec une MII en rémission par induction/favorisation de la cicatrisation des muqueuses chez le patient et maintien de l'intégrité des muqueuses.

5. Un ou plusieurs HMO ou la composition synthétique pour leur utilisation selon la revendication 4, dans lesquels le patient avec une MII est un patient avec une MII modérée à grave qui reçoit un traitement par immunomodulateur.

6. Un ou plusieurs HMO pour leur utilisation selon la revendication 1 ou 3, ou la composition synthétique pour son utilisation selon la revendication 2 ou 3, dans lesquels l'utilisation est la favorisation de la cicatrisation des muqueuses chez un patient ayant une ulcération due à une affection gastro-intestinale inflammatoire.

7. Un ou plusieurs HMO pour leur utilisation selon la revendication 1 ou 3, ou la composition synthétique pour son utilisation selon la revendication 2 ou 3, dans lesquels l'utilisation est l'induction de la cicatrisation des muqueuses chez un patient ayant une ulcération due à une affection gastro-intestinale inflammatoire.

8. Un ou plusieurs HMO pour leur utilisation selon l'une quelconque des revendications 1 ou 3 à 7, ou la composition synthétique pour son utilisation selon l'une quelconque des revendications 2 ou 7, dans lesquels le patient est un patient pédiatrique ou adulte.

9. La composition synthétique pour son utilisation selon l'une quelconque des revendications 2 à 8, qui est une composition nutritionnelle.

10. La composition synthétique pour son utilisation selon l'une quelconque des revendications 2 à 9, comprenant en outre une source de thréonine, de sérine et/ou de proline.

11. Un ou plusieurs HMO pour leur utilisation selon l'une quelconque des revendications 1 ou 3 à 8, ou la composition synthétique pour son utilisation selon l'une quelconque des revendications 2 à 10, dans lesquels les HMO comprennent
- un ou plusieurs HMO de squelette et un ou plusieurs HMO fucosylés,
- un ou plusieurs HMO de squelette et un ou plusieurs HMO sialylés, ou
- un ou plusieurs HMO fucosylés et un ou plusieurs HMO sialylés.

12. Un ou plusieurs HMO pour leur utilisation selon l'une quelconque des revendications 1 ou 3 à 8, ou la composition synthétique pour son utilisation selon l'une quelconque des revendications 2 à 10, dans lesquels les HMO comprennent un ou plusieurs HMO de squelette, un ou plusieurs HMO sialylés et un ou plusieurs HMO fucosylés.

13. Un ou plusieurs oligosaccharides du lait humain (HMO) pour leur utilisation selon l'une quelconque des revendications 1 ou 3 à 8, ou la composition synthétique pour son utilisation selon l'une quelconque des revendications 2 à 10, dans lesquels l'oligosaccharide du lait humain est sélectionné parmi les 2'-FL, 3-FL, DFL, 3'-SL, 6'-SL, LNT, LNnT, LNFP-I et DSLNT.
